# EUROPEAN PATENT SPECIFICATION

(11) **EP 1 902 036 B1**
(45) Date of publication and mention of the grant of the patent: **24.03.2010**
(21) Application number: 06744403.4
(22) Date of filing: 26.05.2006
(51) Int. Cl.: C07D 239/42, C07D 405/06

(54) **PROCESS FOR THE PREPARATION OF ROSUVASTATIN AND INTERMEDIATES**
VERFAHREN ZUR HERSTELLUNG VON ROSUVASTATIN UND INTERMEDIATE
PROCEDE DE PREPARATION DE ROSUVASTATINE ET INTERMEDIAIRES

(30) Priority: 26.05.2005 HU 0500537
(43) Date of publication of application: 26.03.2008
(73) Proprietor: Richter Gedeon Nyrt., 1103 Budapest (HU)
(72) Inventor: FISCHER, János, H-1014 Budapest (HU); SZEMZÖ, Attila, H-1118 Budapest (HU); VUKICS, Krisztina, H-1111 Budapest (HU); ERDELYI, Péter, H-1103 Budapest (HU); SZÖKE, Katalin, H-1105 Budapest (HU); DONÁT, Andrea, H-1102 Budapest (HU)
(74) Representative: Spilgies, Jan-Hendrik
(86) International application number: PCT/HU2006/000049
(87) International publication number: WO 2006/126035

(56) References cited:
- WO-A-00/49014
- WO-A-01/85702
- WO-A-2005/042522

## Description

The present invention relates to a new process for the preparation of a HMG-CoA reductase inhibitor rosuvastatin of formula I 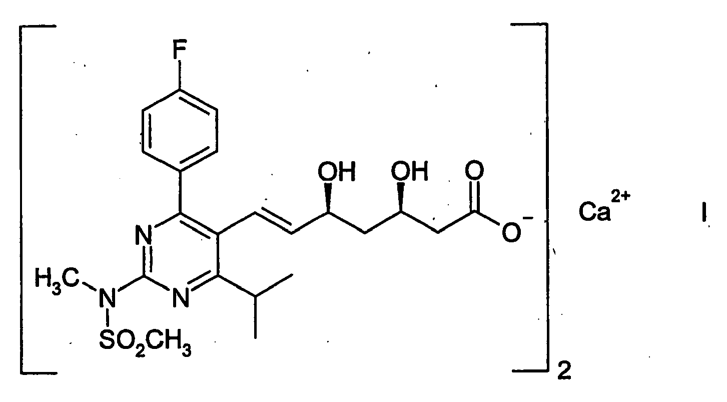 which comprises one of the following processes
a.) reacting a compound of formula II 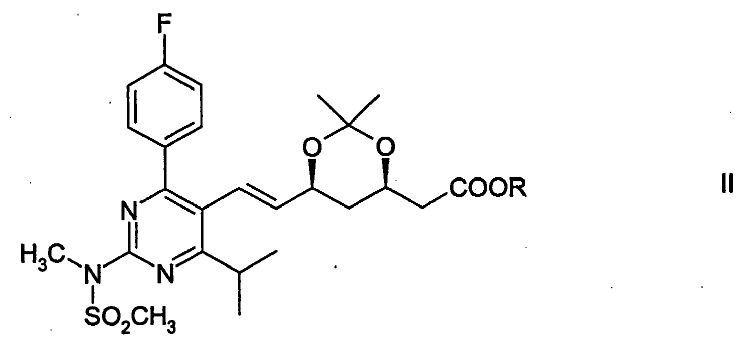 -wherein R represents C₁₋₄ alkyl except tert-butyl group- by alkalic hydrolysis to give a compound of formula III 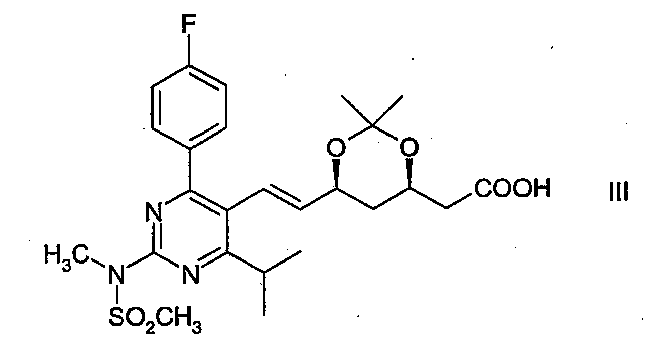 thereafter reacting with an organic or inorganic base to form a salt, eliminating the acetonide group and reacting with calcium-chloride in a base; or
b.) reacting a compound of formula III 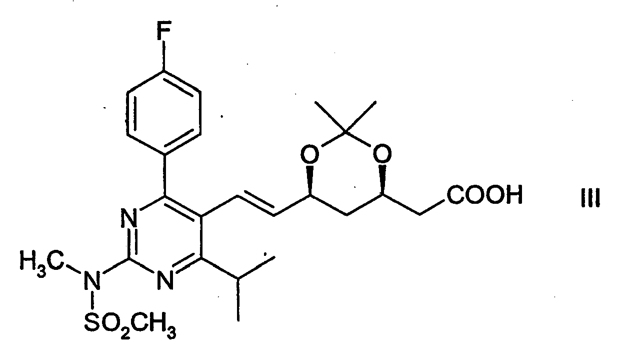 with an organic or inorganic base to form a salt, eliminating the acetonide group and reacting with calcium-chloride in a base; or
c.) reacting a salt of a compound of formula III 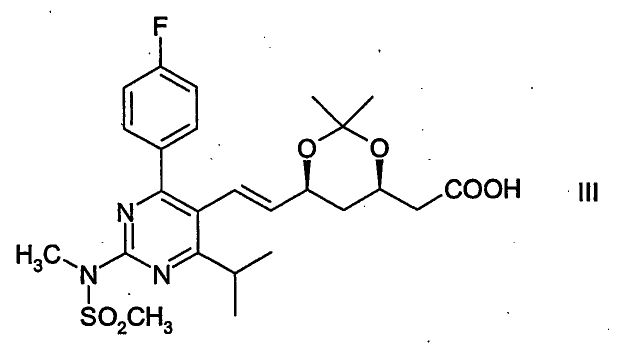 -formed with an organic or inorganic base- by eliminating the acetonide group and reacting with calcium-chloride in a base.

The further subject of the present invention is a compound of formula III and its salts formed by organic and inorganic base.

Rosuvastatin (chemical name: (E)-7-[4-(4-fluorophenyl)-6-isopropyl-2-(methanesulfonyl-methyl-amino)-pyrimidin-5-yl]-3,5-dihydroxy-hept-6-anoic acid calcium salt) and its preparation process is described in European Patent EP 521471. The active ingredient is prepared by the reduction of 7-[4-(4-fluorophenyl)-6-isopropyl-2-[methyl(methylsulfonyl)amino]pyrimidin-5-yl](3R)-3-hydroxy-5-oxo-(E)-heptenoic acid and further steps of preparation. It is not a cost-effective preparation process because the 5S-hydroxy group of the side chain forming heptanoic acid is synthesized at the and of the preparation process, in one of the last steps.

Another preparation process of rosuvastatin is described in International Patent Application WO 00/49014. According to this process tert-butyl-E-(6-{2-[4-(4-fluorophenyl)-6-isopropyl-2-[methyl(methylsulfonyl)amino]pyrimidin-5-yl]-vinyl}-((4R,6S)-2,2-dimethyl[1,3]dioxan-4-yl) acetic acid which is a key intermediate and abbreviated as BEM, is prepared by the reaction of diphenyl-[4-(4-fluorphenyl)-6-isopropyl-2-[methyl(methylsulfonyl)amino]pyrimidin-5-ylmethyl]phosphine oxide and tert-butyl-2-[(4R,6S)-6-formyl-2,2-dimethyl-1,3-dioxan-4-yl] acetic acid.

According to the WO 00/49014 PCT-application the sodium salt of the end product is prepared from BEM by the cleavage of the acetonide protecting group by acid hydrolysis using 0,02 M hydrochloric acid at 40 °C and by the cleavage of the tert-butyl protecting group using 1 M sodium hydroxide at 25 °C. The carboxylic acid is liberated from the sodium salt of rosuvastatin with hydrochloric acid (1 M) at -5 °C at a pH between 3. 4 and 4. 0. The methylamine salt is isolated from carboxylic acid with 40 % methylamine solution in water with a yield of 82 %. The methylamine salt is converted again to sodium salt with 8 % w/w aqueous sodium hydroxide solution and the methyl amine is eliminated with a complicated distillation process. The end product is prepared from the sodium salt by calcium chloride dihydrate. The application does not contain any data about the yield of the end product.

International Patent Application WO 00/49014 describes a long, complicated preparation process containing six-operational steps for the preparation of the pharmaceutical drug.

The disadvantage of the above mentioned process is the use of methylamine, which is unhealthy. The basic character and the volatility of methylamine induce its toxic, mucosa-destroying, mutagen and teratogen effects. (MSDS Data Sheet: http://physchem.ox.ac.uk/MSDS/; NCI Chem Carcinogenesis Res Info System: http://toxnet.nlm.nih.gov/ ; Schardein JL Chemically Induced Birth Defects 1993;2:842-69).

The last part of the preparation -before the separation of the calcium salt- does not contain any possibility for the ideal purification. The first obtained sodium salt can not be purified and the methylamine salt prepared from the carboxylic acid can contaminate the product, because its elimination by distillation is difficult.

The above mentioned disadvantages result the low yields, altogether 60-70 % of the preparation process described in WO 00/49014.

International Patent Application WO 2004/108691 relate to an alternative solution for the preparation of rosuvastatin using the BEM intermediate. The end product is obtained from the ester without the preparation of a salt of the intermediate. The difference compared to the preparation process described in WO 00/49014 PCT-application is that the purification step with methylamine salt is missing and E-7-[4-(4-fluorphenyl)-6-isopropyl-2-[methyl(methylsulfonyl)amino]pyrimidin-5-yl](3R,5S)-3,5-dihydroxyhept-6-enoate salt is prepared directly from the sodium salt, obtained by ester hydrolysis. The sodium salt obtained by this process can not be purified.

Considering to the above mentioned disadvantages our aim was to work out a new, simple process for preparing crystalline intermediates of high purity which enables to prepare rosuvastatin with a high purity and with significantly improved yields.

During our experiments a new compound of formula III 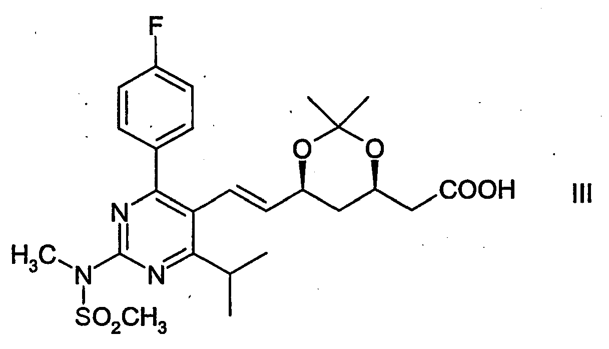 and its salts formed by organic or inorganic base was prepared.

Salts of the new compound of formula III can be prepared with high purity and a yield of 90 %. Salts formed from the compound of formula III with an organic or inorganic base can be prepared by a good yield an it can be easily purified. The crystalline nature of these salts ensures a pure end product, which is acknowledged as an important active ingredient in the pharmaceutical industry. The following bases can be used advantageously for the salt formation: methylamine, diethanolamine, ethanolamine, magnesium sulfate, L-lysine, benzylamine, L(-)-α-methyl-benzylamine or N-methyl-D-glucosamine. Especially preferred salts are diethanolamine, L-lysine and magnesium salts. These bases are knows salt-forming agents at the salt formation of other active ingredients, as well. (P.H.Stahl, C.G.Wermuth (Eds.) Handbook of Pharmaceutical Salts; Properties, Selection and Use, Wiley-VCH, 2002).

The subject of the present invention is a process for the preparation of rosuvastatin of formula I 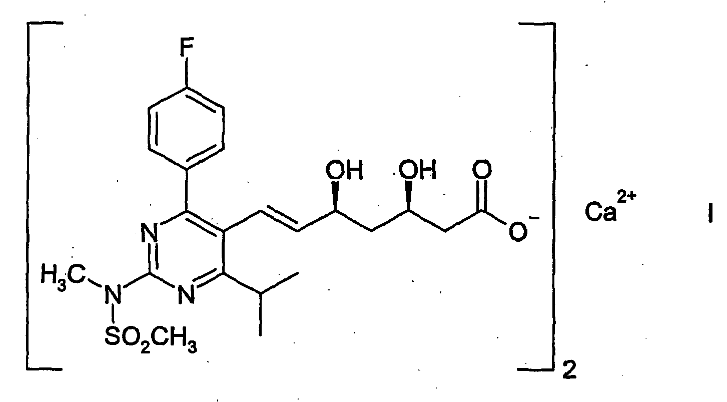 which comprises one of the following processes
b.) reacting a compound of formula II, 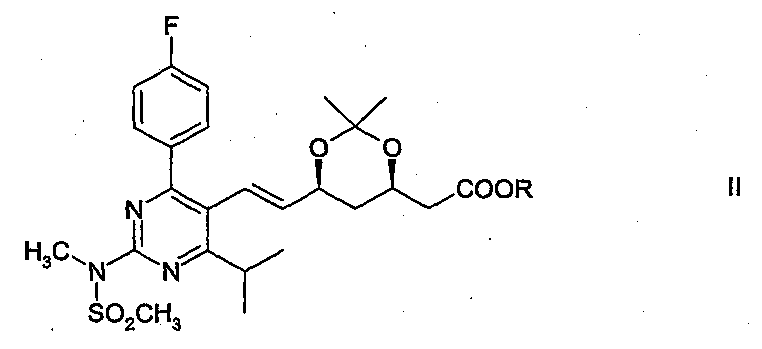 -wherein R represents C₁₋₄ alkyl except tert-butyl group- by alkalic hydrolysis to give a compound of formula III 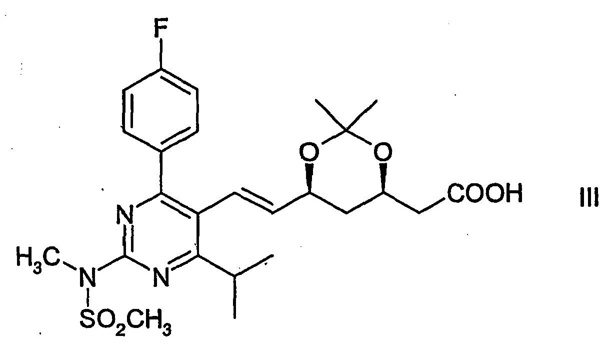 thereafter reacting with an organic or inorganic base to form a salt, eliminating the acetonide group and reacting with calcium-chloride in a base; or
b.) reacting a compound of formula III 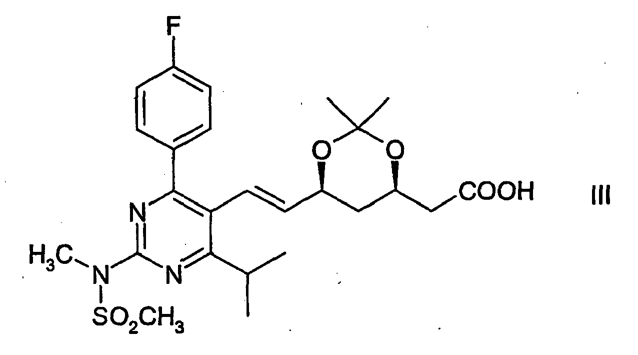 with an organic or inorganic base to form a salt, eliminating the acetonide group and reacting with calcium-chloride in a base; or
c.) reacting a salt of a compound of formula III 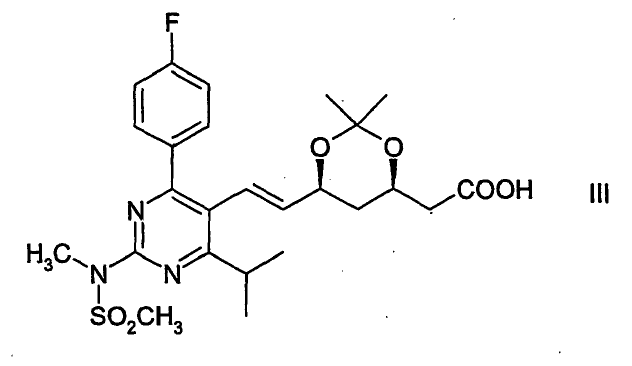 -formed with an organic or inorganic base- by eliminating the acetonide group and reacting with calcium-chloride in a base.

In the preparation of compound of formula I the salt formation is carried out in a hydrous or anhydrous, neutral organic solvent.

In the preparation of compound of formula I the neutral organic solvent is preferably acetonitrile, ethyl-acetate or ethanol.

In the preparation of compound of formula I the salt formation from compound of formula III is carried out with methylamine, diethanolamine, ethanolamine, magnesium sulfate, L-lysine, benzylamine, L(-)-α-methyl-benzylamine or N-methyl-D-glucosamine.

In the preparation of compound of formula I the salt formation is carried out preferably with diethanolamine, L-lysine or magnesium sulfate.

In the preparation of compound of formula I the elimination of the acetonide group is carried out in neutral solvent in the presence of an acid.

In the preparation of compound of formula I the neutral solvent is tetrahydrofuran.

A further subject of the present invention is a compound of formula III 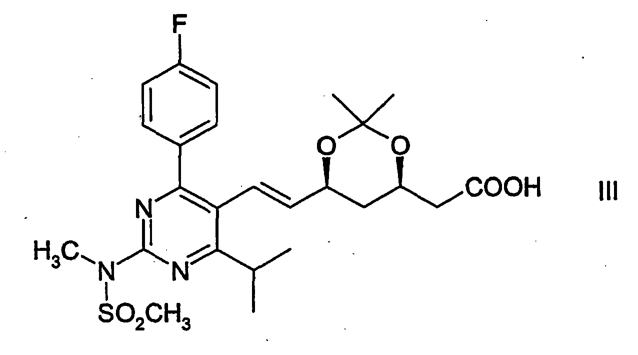 and its salts formed by organic or inorganic base.

A further subject of our invention is a salt of a compound of formula III formed by methylamine, diethanolamine, ethanolamine, magnesium sulfate, L-lysine, benzylamine, L(-)-α-methyl-benzylamine or N-methyl-D-glucosamine.

Other subjects of our invention are (6-{(E)-2-[4-(4-fluorophenyl)-6-isopropyl-2-(methanesulfonyl-methyl-amino)-pyrimidin-5-yl]-vinyl}-(4R,6S)-2,2-dimethyl-[1,3]dioxan-4-yl)-acetic acid-diethanolamine salt, (6-{(E)-2-[4-(4-fluorophenyl)-6-isopropyl-2-(methanesulfonyl-methylamine)-pyrimidin-5-yl]-vinyl}-(4R,6S)-2,2-dimethyl-[1,3]dioxan-4-yl)-acetic acid-L-lysine salt and (6-{(E)-2-[4-(4-fluorophenyl)-6-isopropyl-2-(methanesulfonyl-methylamine)-pyrimidin-5-yl]-vinyl}-(4R,6S)-2,2-dimethyl-[1,3]dioxan-4-yl)-acetic acid-magnesium salt.

Alkylesters of general formula II was prepared according to the process described in International Patent Application WO 00/49014 by the reaction of N-[5-[( diphenylphosphinoil)-methyl]-4-(4-fluorophenyl)-6-isopropyl-pyrimidin-2-yl]-N-methyl-methansulfonamide and 6-formyl-2,2-dimethyl-1,3-dioxan-4-yl-acetic acid in the presence of a strong base.

The preparation of an acid of formula III can be carried out by the alkalic hydrolysis of alkylesters of formula II advantageously in tetrahydrofuran with 1M sodium hydroxide.

Thereafter a new salt is formed from the compound of formula III with organic or inorganic base in hydrous or anhydrous, neutral solvent. The salt formation is carried out preferably with methylamine, diethanolamine, ethanolamine, magnesium sulfate, L-lysine, benzylamine, L(-)-α-methyl-benzylamine or N-methyl-D-glucosamine.

Salts formed from the a compound of formula III can be used for the preparation of calcium-E-7-[4-(4-fluorphenyl)-6-isopropyl-2-[methyl(methylsulfonyl)amino]pyrimidin-5-yl](3R,5S)-3,5-dihidroxihept-6-enoic acid. Acetonide protecting group was cleaved by heating of the above mentioned salts with 1 M hydrochloric acid solution at 80 °C and the filterable amorphous solid product can be prepared from a salt -formed with a strong base, preferably with alkali hydroxide- with calcium-chloride solution. After filtering and drying 90 % yield of the active ingredient was obtained.

The main aspect of the present invention is the preparation of rosuvastatin preparing a new carboxylic acid of formula III from the alkyl ester of formula II and purifying by its salts, which were formed with organic or inorganic base. HMG-CoA reductase inhibitor rosuvastatin can be prepared from these salts with a yield of 90%.

The advantage of the preparation process of the present invention contrary to the preparation process described in the International Patent Application WO 00/49014 is that methylamine salt is not used. Consequently the difficult elimination of methylamine by distillation has not to bee carried out. A further advantage is that the salt prepared from the base used for the salt formation does not contaminate the end product, because the acetonide protecting group is eliminated at the last step by hydrochloric acid (1 M) and the base is located in the aqueous phase as a hydrochloride salt. This preparation does not increases the number of steps of the process, in the contrary, it makes the process simpler because the base is eliminated by simple salt formation instead of distillation.

The new salts prepared according to the invention can be purified easily. Contrary to the process described in International Patent Application WO 2004/108691, wherein the purification of the amorphous end product can not be carried out, our process ensures the preparation of pure calcium-E-7-[4-(4-fluorphenyl)-6-isopropyl-2-[methyl(methylsulfonyl)amino]pyrimidin-5-yl](3R,5S)-3,5-dihidroxihept-6-enoic acid.

### Examples

### Example 1

### (6-{(E)--2-[4-(4-fluorophenyl)-6-isopropyl-2-methanesulfonyl-methyl-amino)-pyrimidin-5-yl]vinyl}(4R,6S)-2,2-dimethyl[1,3]dioxan-4-yl)-acetic acid ethylester

N-[5-[(diphenylphosphinoyl)-methyl]-4-(4-fluorophenyl)-6-isopropyl-pyrimidindin-2-yl]-N-methyl-methanesulfonamide (6.4 g, 14.3 mmol) was dissolved in anhydrous tetrahydrofuran (105 ml) in a flask dried in vacuum at 120 °C in argon atmosphere and 3 g molecular sieve (Merck, 3 A) was added to the solution and it was cooled to -75-78 °C in the mixture of acetone and solid carbone dioxide. Sodium-bis-trimethylsilyl-amide (21.2 ml, 21.1 mmol, 1.0 M/THF) was added dropwise to the reaction mixture over 20 minutes. It was stirred and kept at the same temperature for 10 minutes then 6-formil-2,2-dimethyl-1,3-dioxan-4-yl-acetic acid-ethylester (4.1 g, 18 mmol) in toluol (83 ml) was added to the mixture over 20 minutes.

The reaction mixture was stirred at -75 °C. for 1 hour and the resulted suspension was warmed up to -20 °C over 1 hour. The reaction mixture was added to water (50 ml) in extraction funnel and the flask was washed with toluol-THF mixture (50 ml, ratio= 1:1) and water then it was extracted twice with brine (2 x 50 ml). The residue was solved in ethanol (30 ml) and the product was crystallized at +4 °C. The crystals were filtered and washed in cold ethanol (20 ml) and dried.
Yield: 4.7 g (60%).
Melting point: 191-3 °C.

*(6-{(E)-2-[4-(4-fluorophenyl)-6-isopropyl-2-(methanesulfonyl-methyl-amino)-pyrimidin-5-yl]-vinyl}-(4R,6S)-2,2-dimetyl-[1,3]dioxan-4-yl)-acetic acid-methylester* was prepared according to the method described above in example 1 with the difference that after adding dropwise sodium-bis-trimethylsilil-amide to the parent material 6-formil-2,2-dimethyl-1,3-dioxan-4-yl-acetic acid-methylester was added to the solution.
Yield: 4.8 g (63 %)
Melting point: 130-132 °C

### Example 2

### (6-{(E)-2-[4-(4-fluorophenyl)-6-isopropyl-2-(methanesulfonyl-methyl-amino)-pyrimidin-5-yl]-vinyl}-(4R,6S)-2,2-dimethyl-[1,3]dioxan-4-yl)-acetic acid

(6-{(E)-2-[4-(4-fluorophenyl)-6-isopropyl-2-(methanesulfonyl-methyl-amino)-pyrimidin-5-yl]-vinyl}-(4R,6S)-2,2-dimethyl-[1,3]dioxane-4-yl)-acetic acid ethylester (5.52g, 10 mmol) was dissolved in tetrahydrofuran (10 ml) and 1M sodium hydroxide 30 ml, 30 mmol) was added to the solution by stirring and it was heated to 90-100 °C. The reaction mixture was kept at this temperature until the parent material is transformed (ca. 4-6 hours). (It was analyzed by thin layer chromatography on silica gel in toluol-ethyl acetate (6:1)).

The reaction mixture was diluted with ethyl-acetate (50 ml), the aqueous phase was separated and the organic phase was extracted with water (20 ml) then the organic phase was concentrated in vacuum. Water (100 ml) was added to the residue and the solution was treated with 1 M hydrogen chloride solution by cooling with ice-cold water. The precipitated product was filtered off, washed with cold water (2x 50 ml) and dried at 50 °C.
Yield: 4.9 g (95%)
Melting point: 172-174 °C

### Example 3

### (6-{(E)-2[4-(4-fluorophenyl)-6-isopropyl-2(methanesulfonyl-methyl-amino)-pyrimidin-5-yl]-vinyl}-(4R,6S)-2,2-dimethyl-[1,3]dioxan-4-yl)-acetic acid-methylamine salt

(6-{(E)-2-[4-(4-fluorphenyl)-6-isopropyl-2-(methanesulfonyl-methyl-amino)-pyrimidin-5-yl]-vinyl}-(4R,6S)-2,2-dimethyl-[1,3]dioxan-4-yl)-acetic acid (4.9 g, 9.4 mmol) prepared according to the method described in example 2 was dissolved in acetonitrile and methylamine (1.7 ml, 25 mmol) in 1 M ethanol was added to the solution and it was heated to 80-90 °C and kept at this temperature for 30 minutes. Then the reaction mixture was stirred at room temperature for 10-16 hours and the product was filtered and washed with cold acetonitrile (30 ml) and dried.
Yield: 4.7 g (85%).
Melting point: 190-192°C.

### Example 4

### (6-{(E)-2-[4-(4-fluorophenyl)-6-isopropyl-2-(methanesulfonyl-methyl-amino)-pyrimidin-5-yl]-1-vinyl}-(4R,6S)-2,2-dimethyl-[1,3]dioxan-4yl)-acetic acid diethanolamine salt

(6-{(E)-2-[4-(4-fluorophenyl)-6-isopropyl-2-(methanesulfonyl-methyl-amino)-pyrimidin-5-yl]-vinyl}-(4R,6S)-2,2-dimethyl-[1,3]dioxan-4-yl)-acetic acid- was prepared according to the method described in example 2.

To the solution of (6-{(E)-2-[4-(4-fluorophenyl)-6-isopropyl-2-(methanesulfonyl-methyl-amino)-pyrimidin-5-yl]-vinyl}-(4R,6S)-2,2-dimethyl-[1,3]dioxan-4-yl)-acetic acid (4.9 g, 9.4 mmol) with ethyl-acetate diethanolamine (1.6 ml, 17 mmol) was added dropwise at 80 °C. The reaction mixture was stirred for 10-16 hours at room temperature, and the precipitate was filtered, washed with cold ethyl acetate (30 ml) and dried at room temperature.
Yield: 4.7 g (75%).
Melting point:151-3 °C.

### Example 5

### (6-{(E)-2-[4-(4-fluorophenyl)-6-isopropyl-2-(methanesulfonyl-methyl-amino)-pyrimidin-5-yl]-vinyl}-(4R,6S)-2,2-dimethyl-[1,3]dioxan-4-yl)-acetic acid -ethanolamin salt

A (6-{(E)-2-[4-(4-fluorophenyl)-6-isopropyl-2-(methanesulfonyl-methyl-amino)-pyrimidin-5-yl]-vinyl}-(4R,6S)-2,2-dimethyl-[1,3]dioxan-4-yl)-acetic acid- was prepared according to the method described in example 2.

A solution of (6-{(E)-2-[4-(4-fluorophenyl)-6-isopropyl-2-(methanesulfonyl-methyl-amino)-pyrimidin-5-yl]-vinyl}-(4R,6S)-2,2-dimethyl-[1,3]dioxan-4-yl)-acetic acid (4.9 g, 9.4 mmol) and acetonitrile (110 ml) was heated to 70 °C and 1,1 equivalent ethanolamine was added dropwise to the solution then it was cooled to 50 °C and kept at this temperature for 120 minutes. The reaction mixture was cooled to room temperature over 2 hours and the mixture was cooled in ice-cool water for another 2 hours, filtered and washed with cold acetonitrile (30 ml). The product was dried in vacuum at 40 °C.
Yield: 4.9 g (85%).
Melting point:151-153 °C.

### Example 6

### (6-{(E)-2-[4-(4-fluorophenyl-6-isopropyl-2-(methanesulfonyl-methyl-amino)-pyrimidin-5-yl]-vinyl}-(4R,6S)-2,2-dimethyl-[1,3]dioxan-4-yl)-acetic acid)-magnesium salt

A (6-{(E)-2-[4-(4-fluorophenyl)-6-isopropyl-2-(methanesulfonyl-methyl-amino)-pyrimidin-5-yl]-vinyl}-(4R,6S)-2,2-dimethyl-[1,3]dioxan-4-yl)-acetic acid was prepared according to the method described in example 2.

A solution of (6-{(E)-2-[4-(4-fluorophenyl)-6-isopropyl-2-(methanesulfonyl-methyl-amino)-pyrimidin-5-yl]-vinyl}-(4R,6S)-2,2-dimethyl-[1,3]dioxan-4-yl)-acetic acid (4.9 g, 9.4 mmol) and acetonitrile was concentrated and the residue was dissolved in water (80 ml). Sodium hydroxide (10 ml, 1 M) was added to the solution and a solution of magnesium sulfate dehydrate (1,4 g, 12 mmol) in water (5 ml) was added dropwise to the reaction mixture. It was stirred for 10-16 hours at room temperature, filtered, washed twice with icy water (2x30 ml) and dried.
Yield: 4.8 g (90%).
Melting point: 175-182 °C.

### Example 7

### (6-{(E)-2-[4-(4-fluorophenyl)-6-isopropyl-2-(methanesulfonyl-methyl-amino)-pyrimidin-5-yl]-vinyl}-(4R,6S)-2,2-dimethyl-[1,3]dioxan-4-yl)-acetic acid-L-lysine salt

(6-{(E)-2-[4-(4-fluorophenyl)-6-isopropyl-2-(methanesulfonyl-methyl-amino)-pyrimidin-5-yl]-vinyl}-(4R,6S)-2,2-dimethyl-[1,3]dioxan-4-yl)-acetic acid was prepared according to the method described in example 2.

A solution of (6-{(E)-2-[4-(4-fluorophenyl)-6-isopropyl-2-(methanesulfonyl-methyl-amino)-pyrimidin-5-yl]-vinyl}-(4R,6S)-2,2-dimethyl-[1,3]dioxan-4-yl)-acetic acid (4.9 g, 9.4 mmol) in 80 % ethanol-water 1,1 equivalent L-lysine was added dropwise and the product was filtered, washed with ethanol and dried.
Yield: 4.0 g (60%).
Melting point: 205-208 °C.

### Examples 8-10

The following salt were prepared according to the method described in examples 3-7.

### (6-{(E)-2-[4-(4-fluorophenyl)-6-isopropyl-2-(methanesulfonyl-methyl-amino)-pyrimidin-5-yl]-vinyl}-(4R,6S)-2,2-dimethyl-[1,3]dioxan-4-yl)-acetic acid -benzylamine salt

Yield: 89%;
Melting point: 171-173 °C

### (6-{(E)-2-[4-(4-fluorophenyl)-6-isopropyl-2-(methanesulfonyl-methyl-amino)-pyrimidin-5yl]-vinyl}-(4R,6S)-2,2-dimethyl-[1,3]dioxan-4-yl)-acetic acid -L(-)-α-methylbenzylamine salt

Yield: 84% ;
Melting point: 200-202 °C

### (6-{(E)-2-[4-(4-fluorophenyl)-6-isopropyl-2-(methanesulfonyl-methyl-amino)-pyrimidin-5-yl]-vinyl}-(4R,6S)-2,2-dimethyl-[1,3]dioxan-4-yl)-acetic acid-N-methyl-D-glucosamine salt

Yield: 75%;
Melting point: 170-172 °C

### Example 11

### Amorphous (E)-7-[4-(4-fluorophenyl)-6-isopropyl-2-(methanesulfonyl-methyl-amino)-pyrimidin-5-yl]7-3,5-dihidroxi-hept-6-anoic acid calcium salt (rosuvastatin-Cα)

(6-{(E)-2-[4-(4-fluorophenyl)-6-isopropyl-2-(methanesulfonyl-methyl-amino)-pyrimidin-5-yl]-vinyl}-(4R,6S)-2,2-dimethyl-[1,3]dioxan-4-yl)-acetic acid -methylamine salt (5.52g, 10 mmol) prepared according to the method described in example 3 was suspended in tetrahydrofuran (150 ml) and by stirring hydrochloric acid (1 M, 20 ml, 20 mmol) was added to the suspension and the mixture was heated to 80 °C. Heating was carried out until the parent material was totally transformed (20-30 minutes). (It was analyzed by thin layer chromatography on silica gel in chloroform-methanol (10:1)).

The reaction mixture was cooled to room temperature and ethyl acetate was added to the solution, then by stirring sodium hydroxide solution (2 M, 15 ml, 30 mmol) was added to the reaction mixture. The organic and aqueous phase was separated and the organic phase was extracted with brine (2x20 ml), and concentrated. The residue was dissolved in water (40 ml) and by stirring and cooling with ice-cool water an aqueous solution of calcium-chloride (1.62 g,11 mmol) in 20 ml water was added dropwise to the solution. The reaction mixture was stirred for 1 hour by cooling then it was stirred for 10-15 hours at room temperature. The precipitated product was filtered off and washed with ice-cool water (3x20 ml). The title product was dried in vacuum at 40 °C.
Yield: 4.5 g (90%).
Melting point: 150-180 °C, protracted.

## Claims

1. A process for the preparation of rosuvastatin of formula I 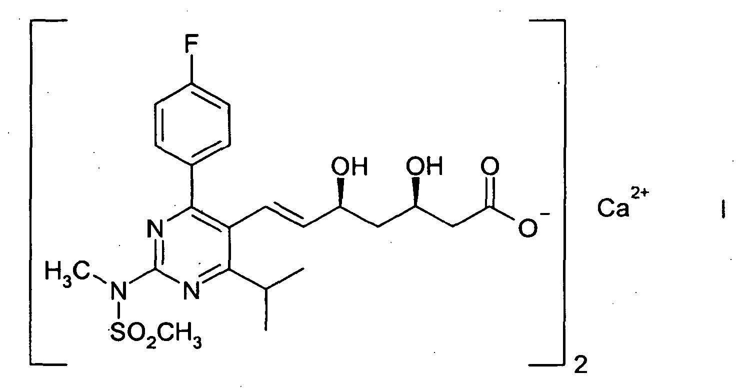 which comprises one of the following processes
a.) reacting a compound of formula II, 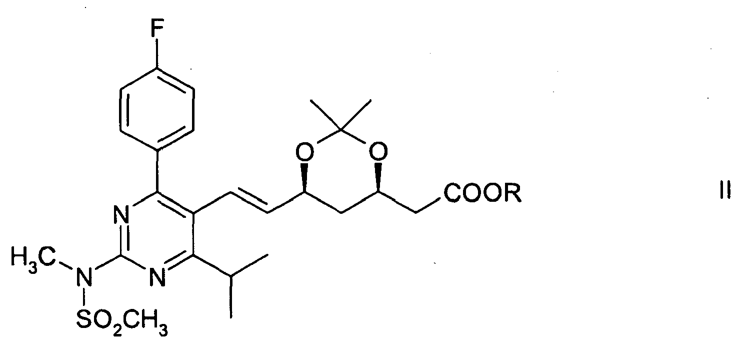 - wherein R represents C1-4 alkyl except tert-butyl group - by alkalic hydrolysis to give a compound of formula III 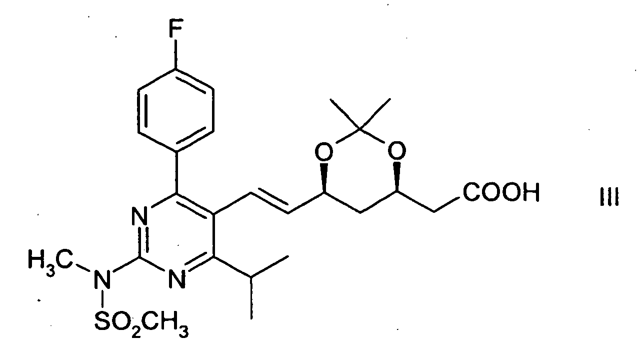 thereafter reacting with an organic or inorganic base to form a salt, eliminating the acetonide group and reacting with calcium-chloride in a base; or
b.) reacting a compound of formula III 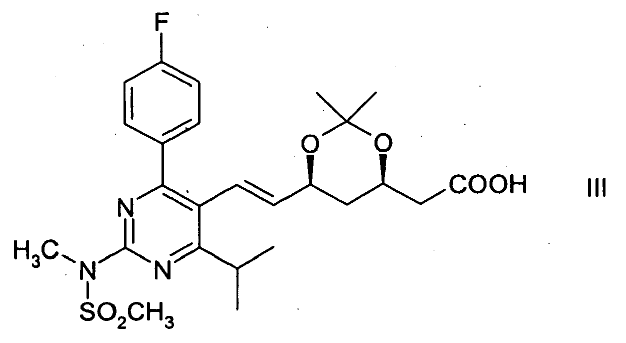 with an organic or inorganic base to form a salt, eliminating the acetonide group and reacting with calcium-chloride in a base; or
c.) reacting a salt of a compound of formula III 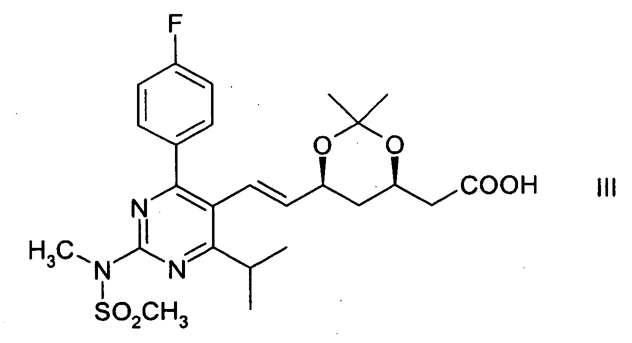 -formed with an organic or inorganic base- by eliminating the acetonide group and reacting with calcium-chloride in a base.

2. A process according to claims 1, wherein the salt formation is carried out with methylamine, diethanolamine, ethanolamine, magnesium sulfate, L-lysine, benzylamine, L(-)-α-methyl-benzylamine or N-methyl-D-glucosamine in a neutral organic solvent,
wherein said neutral organic solvent is acetonitrile, ethyl-acetate, ethanol or tetrahydrofuran.

3. A process according to claims 1-2, wherein the elimination of the acetonide group is carried out in said neutral organic solvent in the presence of an acid.

4. A compound of formula III 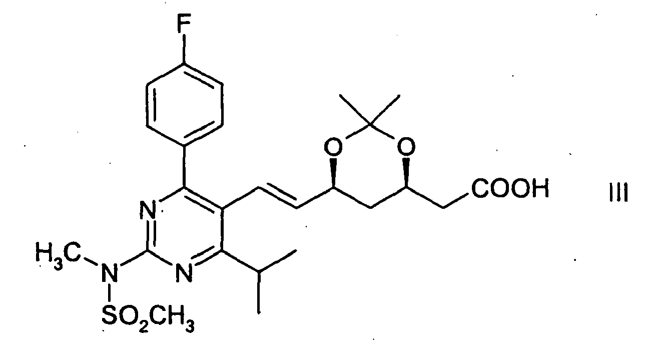 and
a salt formed by a compound of formula III and an organic or inorganic base.

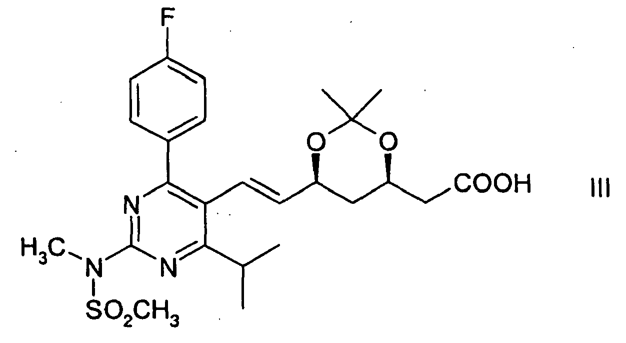

5. A salt of a compound of formula III according to claim 4, wherein the salt is formed by methylamine, diethanolamine, ethanolamine, magnesium sulfate, L-lysine, benzylamine, L(-)-α-methyl-benzylamine or N-methyl-D-glucosamine.

6. A salt of a compound of formula III according to claim 4, which is diethanolamine salt of the (6-{(E)-2-[4-(4-fluorophenyl)-6-isopropyl-2-(methanesulfonyl-methyl-amino)-pyrimidin-5-yl]-vinyl}-(4R,6S)-2,2-dimethyl-[1,3]dioxan-4-yl)-acetic acid.

7. A salt of a compound of formula III according to claim 4, which is L-lysine salt of the (6-{(E)-2-[4-(4-fluorophenyl)-6-isopropyl-2-(methanesulfonyl-methylamine)-pyrimidin-5-yl]-vinyl}-(4R,6S)-2,2-dimethyl-[1,3]dioxan-4-yl)-acetic acid.

8. A salt of a compound of formula III according to claim 4, which is magnesium salt of the (6-{(E)-2-[4-(4-fluorophenyl)-6-isopropyl-2-(methanesulfonyl-methylamine)-pyrimidin-5-yl]-vinyl}-(4R,6S)-2,2-dimethyl-[1,3]dioxan-4-yl)-acetic acid.

## Patentansprüche

1. Verfahren zur Herstellung von Rosuvastatin der Formel (I): 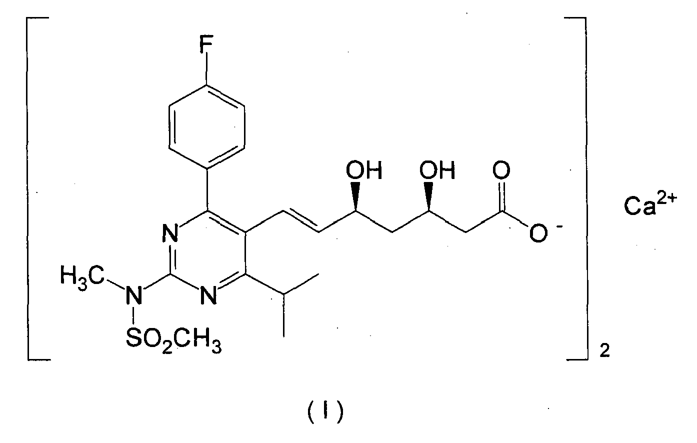 umfassend eines der folgenden Verfahren:
(a) Umsetzen einer Verbindung der Formel (II): 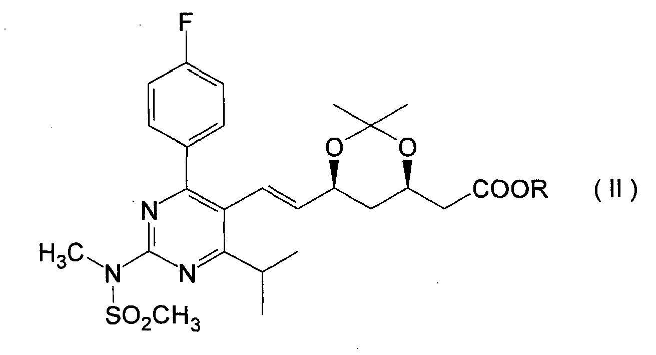 - worin R C₁₋₄-Alkyl, ausser einer tert-Butylgruppe, darstellt - durch alkalische Hydrolyse, um eine Verbindung der Formel (III) zu erhalten: 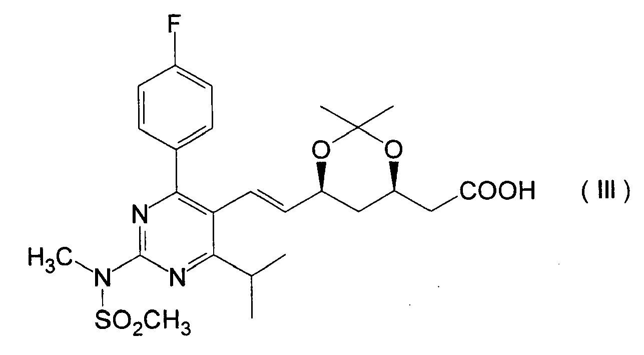 danach Umsetzen mit einer organischen oder anorganischen Base, um ein Salz zu bilden, Entfernen der Acetonidgruppe und Umsetzen mit Calciumchlorid in einer Base; oder
(b) Umsetzen einer Verbindung der Formel (III): 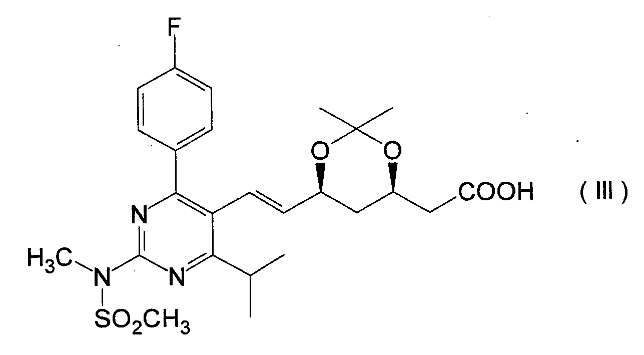 mit einer organischen oder anorganischen Base, um ein Salz zu bilden, Entfernen der Acetonidgruppe und Umsetzen mit Calciumchlorid in einer Base; oder
(c) Umsetzen eines Salzes einer Verbindung der Formel (III): 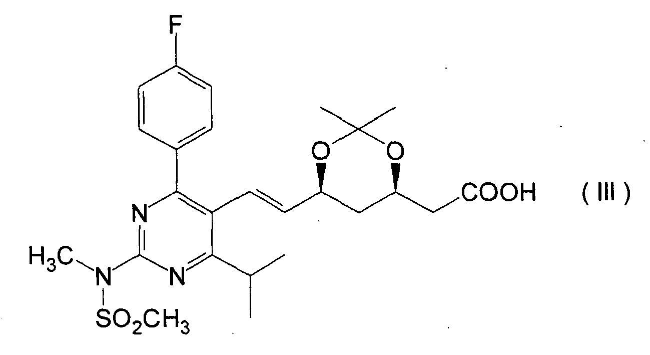 - gebildet mit einer organischen oder anorganischen Base - durch Entfernen der Acetonidgruppe und Umsetzen mit Calciumchlorid in einer Base.

2. Verfahren gemäss Anspruch 1, wobei die Salzbildung mit Methylamin, Diethanolamin, Ethanolamin, Magnesiumsulfat, L-Lysin, Benzylamin, L(-)-α-Methylbenzylamin oder N-Methyl-D-glucosamin in einem neutralen organischen Lösungsmittel durchgeführt wird, wobei das neutrale organische Lösungsmittel Acetonitril, Ethylacetat, Ethanol oder Tetrahydrofuran ist.

3. Verfahren gemäss Ansprüchen 1 bis 2, wobei die Entfernung der Acetonidgruppe in dem neutralen organischen Lösungsmittel in Gegenwart einer Säure durchgeführt wird.

4. Verbindung der Formel (III): 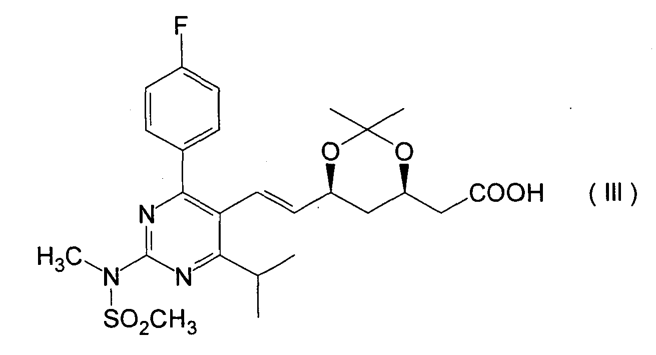 und
ein Salz, das durch eine Verbindung der Formel (III) und eine organische oder anorganische Base gebildet wird.

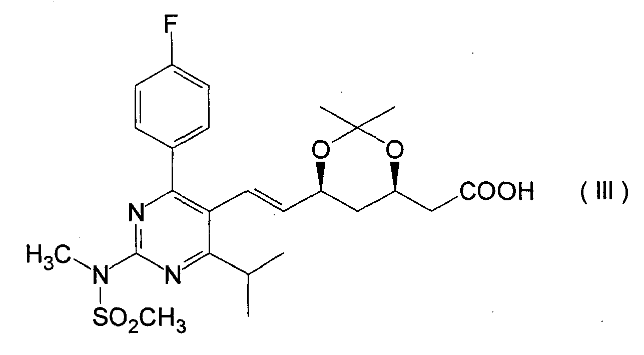

5. Salz oder Verbindung der Formel (III) gemäss Anspruch 4, worin das Salz durch Methylamin, Diethanolamin, Ethanolamin, Magnesiumsulfat, L-Lysin, Benzylamin, L(-)-α-Methylbenzylamin oder N-Methyl-D-glucosamin gebildet wird.

6. Salz oder Verbindung der Formel (III) gemäss Anspruch 4, das ein Diethanolaminsalz der (6-{(E)-2-[4-(4-Fluorphenyl)-6-isopropyl-2-(methansulfonylmethylamino)-pyrimidin-5-yl]-vinyl}-(4R,6S)-2,2-dimethyl-[1,3]dioxan-4-yl)-essigsäure ist.

7. Salz oder Verbindung der Formel (III) gemäss Anspruch 4, das ein L-Lysinsalz der (6-{(E)-2-[4-(4-Fluorphenyl)-6-isopropyl-2-(methansulfonylmethylamino)-pyrimidin-5-yl]-vinyl}-(4R,6S)-2,2-dimethyl-[1,3]dioxan-4-yl)-essigsäure ist.

8. Salz oder Verbindung der Formel (III) gemäss Anspruch 4, das ein Magnesiumsalz der (6-{(E)-2-[4-(4-Fluorphenyl)-6-isopropyl-2-(methansulfonylmethylamino)-pyrimidin-5-yl]-vinyl}-(4R,6S)-2,2-dimethyl-[1,3]dioxan-4-yl)-essigsäure ist.

## Revendications

1. Procédé pour la préparation de rosuvastatine répondant à la formule I : 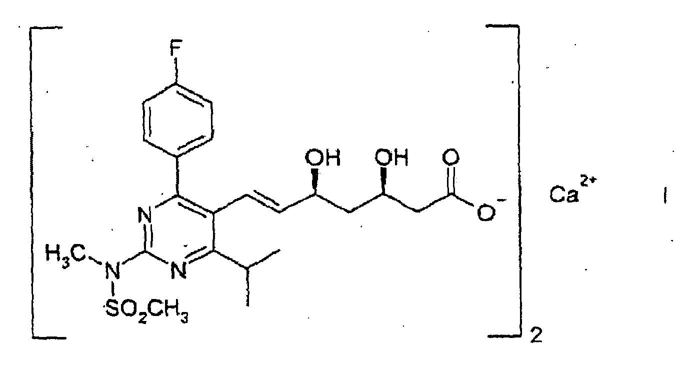 qui comprend un des procédés indiqués ci-après :
a) la mise en réaction d'un composé répondant à la formule II : 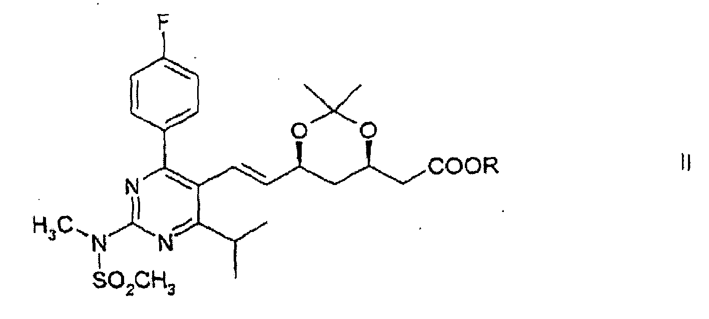 - dans laquelle R représente un groupe alkyle en C₁-C₄, à l'exception d'un groupe tert-butyle - par hydrolyse alcaline pour obtenir un composé répondant à la formule III : 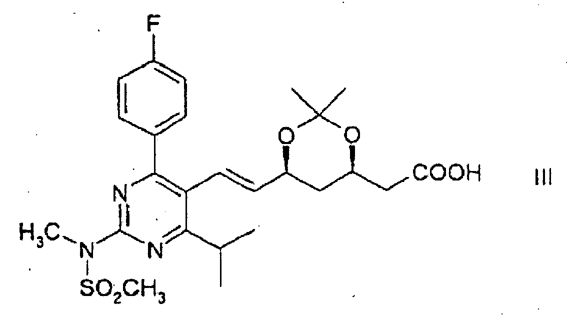 par la mise en réaction ultérieure avec une base organique ou inorganique pour obtenir un sel, l'élimination du groupe acétonide et la mise en réaction avec du chlorure de calcium dans une base ; ou bien
b) la mise en réaction d'un composé répondant à la formule III : 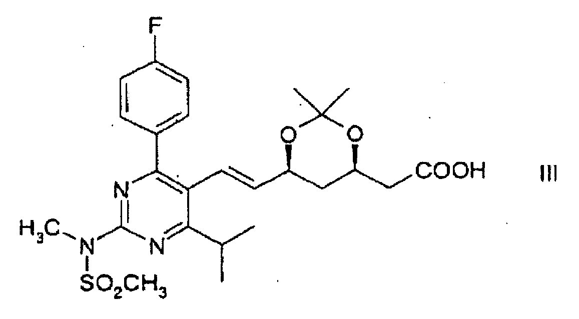 avec une base organique ou inorganique pour obtenir un sel, l'élimination du groupe acétonide et la mise en réaction avec du chlorure de calcium dans une base ; ou bien
c) la mise en réaction d'un sel d'un composé répondant à la formule III : 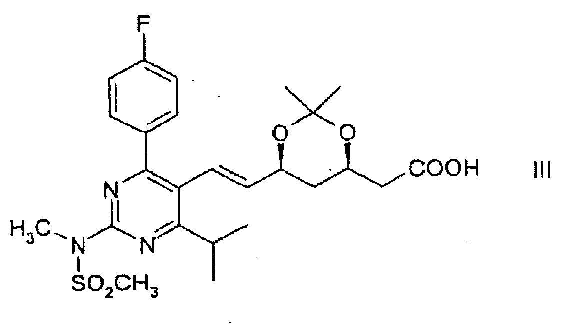 - que l'on obtient avec une base organique ou inorganique - par l'élimination du groupe acétonide et la mise en réaction avec du chlorure de calcium dans une base.

2. Procédé selon la revendication 1, dans lequel la formation du sel est mise en oeuvre avec de la méthylamine, de la diéthanolamine, de l'éthanolamine, du sulfate de magnésium, de la L-lysine, de la benzylamine, de la L(-)-α-méthyl-benzylamine ou de la N-méthyl-D-glucosamine dans un solvant organique neutre, ledit solvant organique neutre étant l'acétonitrile, l'acétate d'éthyle, l'éthanol ou le tétrahydrofuranne.

3. Procédé selon les revendications 1 à 2, dans lequel l'élimination du groupe acétonide est mise en oeuvre dans ledit solvant organique neutre en présence d'un acide.

4. Composé répondant à la formule III 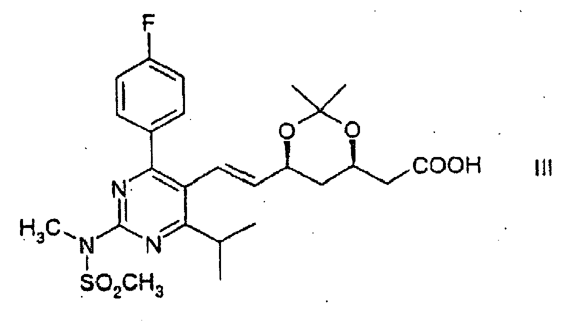 et
sel formé par un composé répondant à la formule III et par une base organique ou inorganique

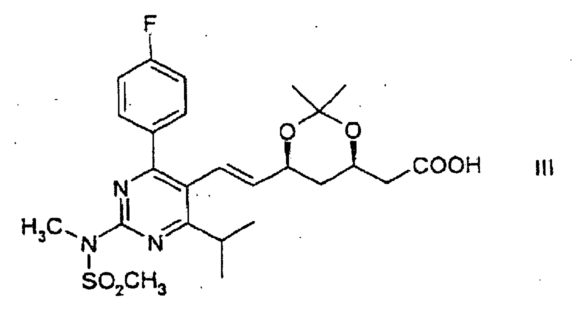

5. Sel d'un composé répondant à la formule III selon la revendication 4, dans lequel le sel est obtenu en utilisant de la méthylamine, de la diéthanolamine, de l'éthanolamine, du sulfate de magnésium, de la L-lysine, de la benzylamine, de la L(-)-α-méthyl-benzylamine ou de la N-méthyl-D-glucosamine.

6. Sel d'un composé répondant à la formule III selon la revendication 4, à savoir le sel de diéthanolamine de l'acide (6-{(E)-2-[4-(4-fluorophényl)-6-isopropyl-2-(méthanesulfonyl-méthyl-amino)-pyrimidin-5-yl]-vinyl}-(4R,6S)-2,2-diméthyl-[1,3]dioxan-4-yl)-acétique.

7. Sel d'un composé répondant à la formule III selon la revendication 4, à savoir le sel de L-lysine de l'acide (6-{(E)-2-[4-(4-fluorophényl)-6-isopropyl-2-(méthanesulfonyl-méthylamine)-pyrimidin-5-yl]-vinyl}-(4R,6S)-2,2-diméthyl-[1,3]dioxan-4-yl)-acétique.

8. Sel d'un composé répondant à la formule III selon la revendication 4, à savoir le sel de magnésium de l'acide (6-{(E)-2-[4-(4-fluorophényl)-6-isopropyl-2-(méthanesulfonyl-méthylamine)-pyrimidin-5-yl]-vinyl}-(4R,6S)-2,2-diméthyl-[1,3]dioxan-4-yl)-acétique.
